(19) Europäisches Patentamt
European Patent Office
Office européen des brevets

(11) Publication number: **0 354 529 A2**

# (12) EUROPEAN PATENT APPLICATION

(21) Application number: 89114637.5

(22) Date of filing: 08.08.89

(51) Int. Cl.[4]: C12P 7/42 , //C07D315/00, (C12P7/42,C12R1:15)

(30) Priority: 09.08.88 US 229959

(43) Date of publication of application: 14.02.90 Bulletin 90/07

(84) Designated Contracting States: AT CH DE FR GB IT LI SE

(71) Applicant: EASTMAN KODAK COMPANY (a New Jersey corporation)
343 State Street
Rochester New York 14650(US)

(72) Inventor: Green, Frederick Richard III
c/o Eastman Kodak Co. Pat. Dept. 343 State Street
Rochester New York 14650(US)
Inventor: Goodhue, Charles Thomas
c/o Eastman Kodak Co. Pat. Dept. 343 State Street
Rochester New York 14650(US)
Inventor: Olyslager, Robert James
c/o EeastmanKodak Co. Pat Dept. 343 State Street
Rochester New York 14650(US)

(74) Representative: Brandes, Jürgen, Dr. et al
Wuesthoff & Wuesthoff Patent- und Rechtsanwälte Schweigerstrasse 2
D-8000 München 90(DE)

## (54) Chiral hydroxycarboxylic acids from prochiral diols.

(57) A method for the production of chiral hydroxycarboxylic acids from prochiral diols is disclosed. The method involves the stereospecific oxidation of the diol to the acid in the presence of a biocatalyst that is derived from Corynebacterium oxydans.

EP 0 354 529 A2

## CHIRAL HYDROXYCARBOXYLIC ACIDS FROM PROCHIRAL DIOLS

In the preparation of many compounds, it is desirable to introduce a chiral center into the compound. Such sterospecific compounds are important for example, because only one stereo isomer of many compounds is biologically active. Methods for the introduction of a chiral center into a compound which can be used directly or as the precursor to another compound are therefore commercially important. The preparation of the stereospecific carboxylic acids would be an example of such a method.

In Jakovac et al. Enzymes in Organic Synthesis. 24. Preparations of Enantiomerically Pure Chiral Lactones via Stereospecific Horse Liver Alcohol Dehydrogenase Catalyzed Oxidations of Monocyclic Meso Diols; J. Am. Chem. Soc. 1982, 104, 4659-4655 there is disclosed the enzymatic preparation of the types of compounds produced by the present invention. However, the enzyme that is used, horse liver alcohol dehydrogenase, requires the cofactor $NAD^+$. Not only is this cofactor expensive, but the reaction sequence must be designed to recycle the NADH that is produced.

The compounds that are produced are quite useful. For example, stereospecific monoacids of meso-bis(hydroxymethyl)cycloalkanes are useful intermediates in the preparation of pyrethrins, such as permethrin and chrysanthemic acid, which are useful agricultural insecticides.

In US Patent 3,558,431 there is described a method for the oxidation of organic compounds using a Corynebacterium which is the preferred bacterium used in the present invention. In the method of this patent, pentaerythritol is converted to tris(hydroxymethyl)acetic acid by fermentation with the organism. No chiral compounds are disclosed and any one of the hydroxy groups in the starting compound can be oxidized. While this reference teaches the oxidation of alcohols using the same catalyst as is used herein, it is not obvious that the reaction would be stereospecific using prochiral compounds so that compounds with excellent enantiomeric excess (e.e.) could be produced. It is just as likely that a racemic mixture would result. It is also likely that if the starting material were a diol, the oxidation would proceed to the diacid. This same microorganism is used in a similar process described in U. S. Patent 3,642,581. (The microorganism in these references is referred to as a Flavobacterium. It is now believed to be a Corynebacterium.)

Thus, there is a continuing need to provide an improved method for producing chiral monocarboxylic acids.

According to the present invention there is provided a method for making chiral hydroxycarboxylic acids from prochiral diols, said method comprising the step of oxidizing said diol in the presence of a catalyst derived from Corynebacterium oxydans.

The method of the present invention is useful for the oxidation of a wide variety of prochiral diols. Useful diols can be represented by the formula;

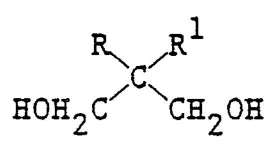

wherein

R and R′ are different and are selected from the group consisting of hydrogen; substituted or unsubstituted alkyl, such as methyl, propyl, chlorobutyl, isopropyl and t-butyl, alkoxy, such as methoxy, propoxy, etc., substituted or unsubstituted benzyl, such as p-chlorobenzyl etc., substituted or unsubstituted aryl, such as phenyl, tolyl, naphthyl and substituted or unsubstituted heterocyclic, such as pyridyl or chloropyridyl.

Other useful diols that can be oxidized according to the present invention include compounds of the formula;

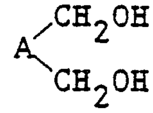

wherein A is a substituted or unsubstituted carbocyclic ring and the hydroxymethyl groups are in 1,2-cis positions, e.g., 1,1-dimethyl-cis-2,3-bishydroxymethylcyclopropane or cis-4, 5-bishydroxymethyl-1-cyclohexene.

The biocatalyst that is used in the present method is derived from Corynebacterium oxydans. By "derived from", we mean that any composition that is made from this species of microorganism that catalyzes the oxidation reaction can be used. Useful compositions include the culture medium containing the cells, the recovered cells themselves or extracts from the cells which include the necessary catalytic activity. The method need not be carried out in the presence of viable cells as in a fermentation. The composition is used as the catalyst in the reaction.

The isolation, maintenance and characterization of a typical Corynebacterium oxydans is described in previously mentioned U. S. Patent 3,558,431. This particular strain, ATCC No. 21,245, is useful in the practice of the present invention. Other strains of this species can also be used, for example ATCC 53,586 and 53,587 both deposited according to the provisions of the Budapest Treaty. Corynebacterium oxydans 53,586 is the currently preferred strain.

In contrast to the method of Jakovak et al mentioned above, the process of the present invention is very simple. The starting diol is simply placed in a suspension of the biocatalyst in water. Oxygen in the water serves as the oxidizing agent and the diol is converted to the desired optically active carboxylic acid. The reaction mixture can contain a small amount of a buffer such as calcium carbonate.

The concentration of the diol in water can vary widely, depending on its solubility in water. The concentration is typically between about 10mM and 500mM. The amount of biocatalyst is also widely variable. Generally it is present in an amount of 0.1-10%, preferably 1%.

The reaction conditions are not critical. The temperature is preferably between about 20° and 50°C. The pH is preferably between about 5 and 9. The reaction time can vary quite widely and due to the mild nature of the reaction conditions can be quite long. Reaction times between about 18h and 72h are typical.

In the examples which follow, the yield is calculated based on the moles of starting diol. The enantiomeric excess or "e.e." is determined from the $H^1$-n.m.r. spectra of the Mosher's esters (J. Org. Chem. 34, 2543:1969) of the free hydroxyl groups. (The acids that are produced according to the invention are esterified for this determination.)

Recovery of the desired compound from the organic reaction medium is by conventional methods. Where the compound is to be used as an intermediate for another compound, recovery may not be necessary.

## Preparation of Corneybacterium oxydans

Corneybacterium oxydans (ATCC 53586) was grown as follows:

1700 mL of a culture medium was prepared from pentaerythritol (34 g), acetic acid (3.4 g), yeast extract (17 g), potassium hydrogen phosphate (3.4 g), and 17 mL of a salt solution prepared from a 1 L solution of $MgSO_4.7H_2O$ (0.25 g), $MnSO_4.7H_2O$ (0.17 g), $FeSO_4.\ 7H_2O$ (0.028 g), NaCl (0.0006 g), $CaCl_2.2H_2O$ (0.001 g), and $ZnSO_4.7H_2O$ (0.006 g). The pH was adjusted to 7 with potassium hydroxide.

The pure culture was used to inoculate two 25 mL samples of the above sterilized culture medium, which were then shaken at 30° C at 250 RPM for about 15 hours.

The flasks were combined and used to inoculate 500 mL of the above sterilized culture medium, which was then shaken at 30° C at 125 RPM for 24 hours.

The solution was then centrifuged at 9000 RPM for 20 minutes, and the resulting cell pellet was frozen at -20°C or lyophilized.

The following examples are presented for a further understanding of the invention.

## Example 1:

Diol 1a (250 mg) was dissolved in water (25 mL) containing a suspension of $CaCO_3$ (250 mg) and Corynebacterium oxydans (ATCC 53,586, 250 mg of a cell paste containing ca. 20% dry weight cells) and shaken on a rotary shaker at 250 rpm for 24 hours. The product was isolated by standard techniques to give a 60% yield of hydroxyacid 1b. This was converted to the methyl ester for determination of the optical purity via its Mosher's ester. The absolute stereochemistry was determined by comparison of the methyl ester acetate with a sample of known stereochemistry.

In a similar manner, diols 2a and 3a were converted to their corresponding hydroxyacids 2b and 3b. The results are shown in Table I:

TABLE I

| | | | | Time | Yield | %ee |
|---|---|---|---|---|---|---|
| 1a | 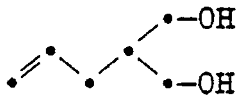 | 1b | 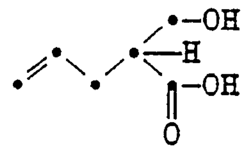 | 24 hr. | 70% | >97% |
| 2a | 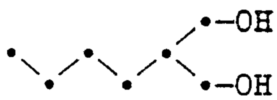 | 2b | 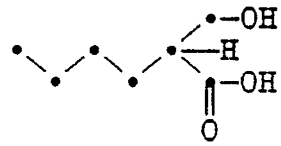 | 48 hr. | 65% | >97% |
| 3a | 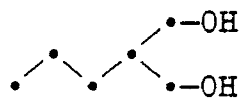 | 3b | 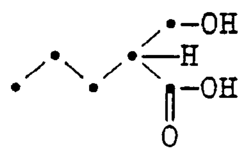 | 24 hr. | 71% | >97% |

Example 2:

Diol 4a (250 mg) was dissolved in water (25 mL) containing a suspension of $CaCO_3$ (250 mg) and Corynebacterium oxydans (ATCC 53586, 250 mg of a cell paste containing ca. 20% dry weight cells) and shaken on a rotary shaker at 250 rpm for 48 hours. After removal of the cells, the reaction medium was acidified to pH 2 with concentrated HCl to effect lactonization of the hydroxyacid. The lactone 4b was isolated by standard techniques in 60% yield. The enantiomeric purity and absolute configuration were determined by comparison of its optical rotation with known values ([α]- 55.8° (c 5.25 $CHCl_3$) 1R,5S).

TABLE II

| | | | | Time | Yield | %ee |
|---|---|---|---|---|---|---|
| 4a | 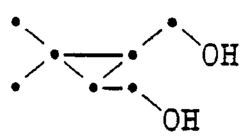 | 4b | 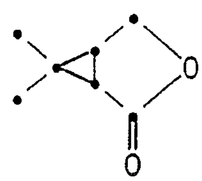 | 48 hr. | 55% | 86% |

**Claims**

1. A method for making chiral hydroxycarboxylic acids from prochiral diols, said method comprising the step of oxidizing said diol in the presence of a catalyst derived from Corynebacterium oxydans.

2. A method according to claim 1 wherein said diol is represented by the formula:

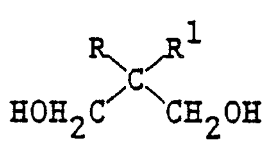

wherein
R and R′ are different and are selected from the group consisting of hydrogen; substituted or unsubstituted alkyl, alkoxy, substituted or unsubstituted benzyl, substituted or unsubstituted aryl and substituted or unsubstituted heterocyclic.

3. A method according to claim 1 wherein said diol is represented by the formula:

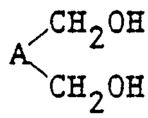

wherein A is a substituted or unsubstituted carbocyclic ring and the hydroxymethyl groups are in the 1,2-cis positions.

4. A method according to claim 1 wherein said catalyst is derived from *Corynebacterium oxydans*, ATCC 53,586.